# EUROPEAN PATENT APPLICATION

(11) **EP 4 588 926 A1**
(43) Date of publication of application: **23.07.2025**
(21) Application number: 24152641.7
(22) Date of filing: 18.01.2024
(51) Int. Cl.: C07F 15/00, B01J 31/00, C07C 6/04, C07C 67/347

(54) **ORGANOMETALLIC COMPOUNDS**

(71) Applicant: Umicore AG & Co. KG, 63457 Hanau-Wolfgang (DE)
(72) Inventor: STOIANOVA, Diana, Monrovia (US); DOPPIU, Angelino, 63500 Seligenstadt (DE); JOHNS, Adam, Claremont (US)
(74) Representative: Clauswitz, Kai-Uwe Wolfram

(57) **Abstract**

an olefin metathesis catalyst represented by the structure of **Formula (I)** 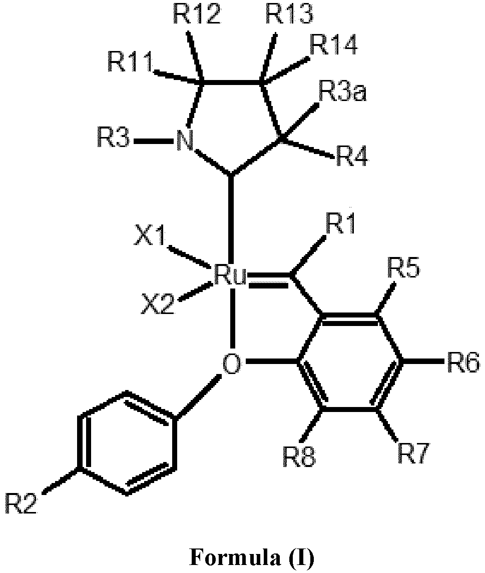 wherein:
R1 is hydrogen or alkyl;
R2 is selected from hydrogen, straight chain alkyl, branched chain alkyl, cycloalkyl, alkoxy, alkylthio, silyloxy, alkylamino, dialkylamino, aryl, aryloxy, heterocyclic or an electron-withdrawing group;
X1 and X2 are the same or different anionic ligands and/or are linked together to form a ring or a ring system;
R3 is adamantyl, unsubstituted phenyl or substituted phenyl;
R3a is alkyl or aryl; or together with R4 can form a cycloalkyl or heterocyclic ring; and
R4 is alkyl or aryl; or together with R3a can form a cycloalkyl or heterocyclic ring;
R11, R12, R13 and R14 are independently hydrogen, unsubstituted alkyl, substituted alkyl, unsubstituted cycloalkyl, substituted cycloalkyl, unsubstituted aryl, substituted aryl, unsubstituted heteroaryl, substituted heteroaryl, unsubstituted aralkyl, substituted aralkyl, unsubstituted heteroaralkyl or substituted heteroaralkyl;
R5, R6, R7 and R8 are, independently from each other, selected from hydrogen, straight chain or branched alkyl groups, alkoxy, alkylthio, silyloxy, alkylamino, or electron-withdrawing groups.

## Description

Ruthenium carbene complexes used to catalyze olefin metathesis reactions are known from the prior art and have gained increasing importance over the past decades. Such olefin metathesis reactions comprise a metal-catalyzed rearrangement of carbon-carbon double bonds and is useful for the production of complex organic compounds, e.g. in synthesis of natural substances, and polymers. Such reactions are often limited by its initiation rate, which results in rapid olefin metathesis reactions requiring higher temperatures or more rapidly initiating catalysts.

Ruthenium carbene complexes, often also referred to as Ruthenium catalysts, are frequently used and highly suitable for catalyzing these reactions due to high stability and tolerance of functional groups. Their stability could be increased, together with their reactivity by various alterations of their ligands. Hoveyda-Grubbs type catalysts are known from the prior art and are typically characterized by a 2-isopropoxy-group at a benzylidene ligand, wherein the oxygen atom is bound to the ruthenium atom in a chelating manner; see formula (a).

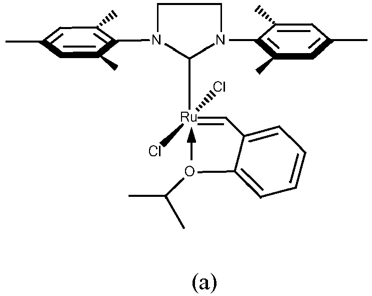

This metathesis catalyst is shown in WO 02/14376 A2. A catalyst loading of about 1 mol-% to 5 mol-% and moderate to high reaction temperatures result in reaction times up to 44 hours to obtain a sufficient yield of the metathesis reaction product, dependent on the substrate used.

Later modifications have been made on the benzylidene group by experimenting with electron-withdrawing and electron-donating groups (Y, Z) at the 4- and 5-position of the benzene ring which also influences the Ru=CHR bond as well (see formula (b)).

Formula (b) is an example of ruthenium carbene complexes, which are shown in Tzur, E., Szadkowska, A., Ben-Asuly, A., Makal, A., Goldberg, I., Wozniak, K., Grela, K., Lemcoff, N. G., Chem. Eur. J. 2010, 16, 8726-8737. The catalysts show activity both for ring-closing metathesis and cross metathesis.

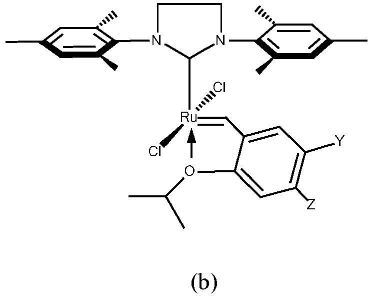

Experimental data showed sufficient product yields with catalyst loadings of between 1 to 2.5 mol-%. Lower yields were obtained in more challenging metathesis reactions such as cross metathesis.

Various olefin metathesis catalysts are shown by Diesendruck, C. E., Tzur, E., Ben-Asuly, A., Goldberg, I., Straub, B. F., Lemcoff, N. G., Inorg. Chem. 2009, 48, 10819-10825, suggesting nitrogen, sulfur, selenium and phosphorus as chelating atoms.

Zukowska, K., Szadkowska, A., Pazio, A. E., Wozniak, K., Grela, K., Organometallics 2012, 31, 462-469 shows pyridine-based ruthenium catalysts containing an amine chelating ligand bearing a chelating nitrogen atom. High temperatures, reaction times of hours to days as well as moderate to high catalyst loading were necessary for sufficient yield of the metathesis reaction products.

Peek, L. H., Savka, R. D., Plenio, H., Chem. Eur. J. 2012, 18, 12845-12853 show *N*-chelated Grubbs-Hoveyda type catalysts and are illustrated in formula (c) and (d) below. Their preparation is expensive and time-consuming due to fact that the precursors must be obtained by a laborious and expensive methods comprising several steps.

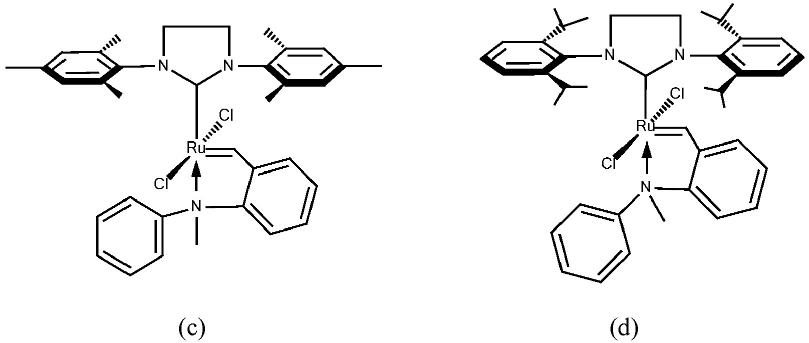

In summary, there is still a need for novel ruthenium metathesis catalysts because a rate of catalyst initiation can be a limiting factor for certain metathesis transformations catalyzed by stereoretentive and cyclic alkyl amino carbene (CAAC) bearing catalysts.

This problem is solved by substituting the chelating o-isopropoxy moiety with a phenoxy group in the ortho position. Surprisingly, this change increases the rate of catalyst activation and improves reaction kinetics.

Consequently, the problem is solved by an olefin metathesis catalyst represented by the structure of **Formula (I)** 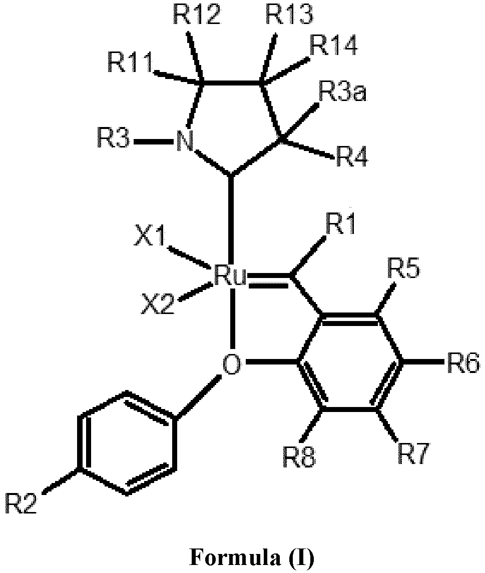 wherein:
R1 is hydrogen or alkyl;
R2 is selected from hydrogen, straight chain alkyl, branched chain alkyl, cycloalkyl, alkoxy, alkylthio, silyloxy, alkylamino, dialkylamino, aryl, aryloxy, heterocyclic or an electron-withdrawing group;
X1 and X2 are the same or different anionic ligands and/or are linked together to form a ring or a ring system;
R3 is adamantyl, unsubstituted phenyl or substituted phenyl;
R3a is alkyl or aryl; or together with R4 can form a cycloalkyl or heterocyclic ring; and
R4 is alkyl or aryl; or together with R3a can form a cycloalkyl or heterocyclic ring;
R11, R12, R13 and R14 are independently hydrogen, unsubstituted alkyl, substituted alkyl, unsubstituted cycloalkyl, substituted cycloalkyl, unsubstituted aryl, substituted aryl, unsubstituted heteroaryl, substituted heteroaryl, unsubstituted aralkyl, substituted aralkyl, unsubstituted heteroaralkyl or substituted heteroaralkyl;
R5, R6, R7 and R8 are, independently from each other, selected from hydrogen, straight chain or branched alkyl groups, alkoxy, alkylthio, silyloxy, alkylamino, or electron-withdrawing groups.

### Short Description of the Invention

1. An olefin metathesis catalyst represented by the structure of **Formula (I)** 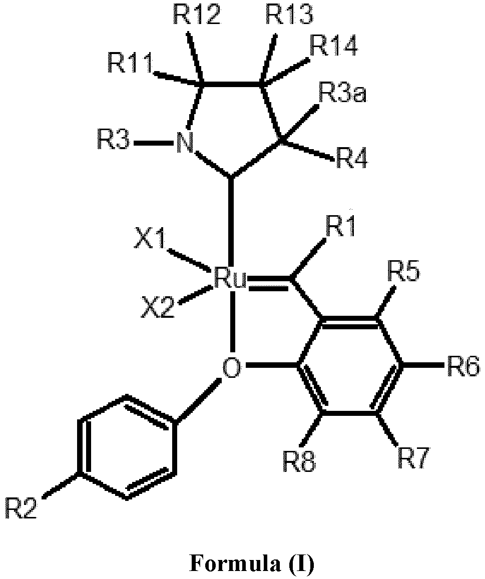 wherein:
   R1 is hydrogen or alkyl;
   R2 is selected from hydrogen, straight chain alkyl, branched chain alkyl, cycloalkyl, alkoxy, alkylthio, silyloxy, alkylamino, dialkylamino, aryl, aryloxy, heterocyclic or an electron-withdrawing group;
   X1 and X2 are the same or different anionic ligands and/or are linked together to form a ring or a ring system;
   R3 is adamantyl, unsubstituted phenyl or substituted phenyl;
   R3a is alkyl or aryl; or together with R4 can form a cycloalkyl or heterocyclic ring; and
   R4 is alkyl or aryl; or together with R3a can form a cycloalkyl or heterocyclic ring;
   R11, R12, R13 and R14 are independently hydrogen, unsubstituted alkyl, substituted alkyl, unsubstituted cycloalkyl, substituted cycloalkyl, unsubstituted aryl, substituted aryl, unsubstituted heteroaryl, substituted heteroaryl, unsubstituted aralkyl, substituted aralkyl, unsubstituted heteroaralkyl or substituted heteroaralkyl;
   R5, R6, R7 and R8 are, independently from each other, selected from hydrogen, straight chain or branched alkyl groups, alkoxy, alkylthio, silyloxy, alkylamino, or electron-withdrawing groups.
2. The olefin metathesis catalyst of item 1, wherein R1 is hydrogen.
3. The olefin metathesis catalyst of item 1 to 2, wherein R2 is selected from hydrogen, C1-C30-alkyl, C1-C30-alkoxy, C1-C30-alkylthio, C1-C30-silyloxy, C1-C30-alkylamino, C1-C30-dialkylamino, C5-C30-aryl, CS-C30-aryloxy, C5-C30-heterocyclic or an electron-withdrawing group.
4. The olefin metathesis catalyst of item 1 to 3, wherein X1 and X2 are the same or different and halogen or X1 and X2 are a heteroatom and are connected by a C1-C5 bridge that may be saturated or unsaturated and can be part of an aromatic or aliphatic ring or ring system.
5. The olefin metathesis catalyst of item 1 to 4, wherein R3 is adamantyl or a structure of the formula 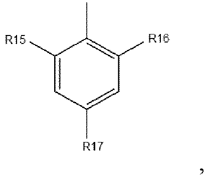 wherein R15, R16 and R17 are, independently of each other, are hydrogen, C1-C30-alkyl, C5-C30-aryl or a C1-C30-alkyl that is substituted with C5-C30-aryl.
6. The olefin metathesis catalyst of item 1 to 5, wherein R3a is C1-C30-alkyl, C5-C30-aryl or together with R4 can form a four to fourteen membered cycloalkyl or heterocyclic ring system with the carbon atom to which they are attached.
7. The olefin metathesis catalyst of item 1 to 6, wherein R4 is is C1-C30-alkyl, C5-C30-aryl or together with R3a can form a four to fourteen membered cycloalkyl or heterocyclic ring system with the carbon atom to which they are attached.
8. The olefin metathesis catalyst of item 1 to 7, wherein R5, R6, R7 and R8 are, independently from each other, selected from hydrogen, straight chain or branched C1-C30-alkyl, C1-C30-alkoxy, C1-C30-alkylthio, C1-C30-silyloxy, C1-C30-alkylamino, or electron-withdrawing groups selected from halogen atoms, trifluormethyl (-CF₃), nitro (-NO₂), sulfinyl (-SO-), sulfonyl (-SO₂-), formyl (-CHO), C1-C30-carbonyl, C1-C30-carboxyl, C1-C30-alkylamido, C1-C30-aminocarbonyl, nitrile (-CN) or C1-C30-sulfonamide.
9. The olefin metathesis catalyst of item 1 to 8, wherein R11, R12, R13 and R14 are independently hydrogen, unsubstituted C1-C30 alkyl, substituted C1-C30 alkyl, unsubstituted C4-C30 cycloalkyl, substituted C4-C30 cycloalkyl, unsubstituted C5-C30 aryl, substituted C5-C30 aryl, unsubstituted C5-C30 heteroaryl, substituted C5-C30 heteroaryl, unsubstituted C6-C30 aralkyl, substituted C6-C30 aralkyl, unsubstituted C6-C30 heteroaralkyl or substituted C6-C30 heteroaralkyl.
10. The olefin metathesis catalyst of item 1 to 9, wherein R2 is selected from hydrogen, straight chain or branched alkyl groups including C1-C10-alkyl, C1-C10-alkoxy, C1-C10-alkylthio, C1-C10-silyloxy, C1-C10-alkylamino, C1-C10-dialkylamino, C6-C14-aryl, C6-C14-aryloxy, C6-C14-heterocyclic or an electron-withdrawing group.
11. The olefin metathesis catalyst of item 1 to 10, wherein X1 and X2 are independently selected from Cl, Br, I or F or are independently oxygen or sulfur linked together by a hydrocarbon bridge being part of an aromatic ring or ring system independently.
12. The olefin metathesis catalyst of item 1 to 11, wherein R3 is 1-adamantyl, 2-adamantyl or a structure of the formula 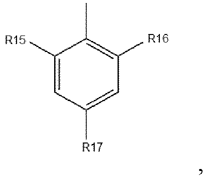 wherein R15, R16 and R17 are, independently of each other, are hydrogen, C1-C12-alkyl, C6-C14-aryl or a C1-C12-alkyl that is substituted with C6-C14-aryl.
13. The olefin metathesis catalyst of item 1 to 12, wherein R3a is C1-C12-alkyl, C6-C14-aryl or together with R4 can form a five to ten membered cycloalkyl or heterocyclic ring system with the carbon atom to which they are attached.
14. The olefin metathesis catalyst of item 1 to 13, wherein R4 is C1-C12-alkyl, C6-C14-aryl or together with R3a can form a five to ten membered cycloalkyl or heterocyclic ring system with the carbon atom to which they are attached.
15. The olefin metathesis catalyst of item 1 to 14, wherein R5, R6, R7 and R8 are, independently from each other, selected from hydrogen, straight chain or branched C1-C10-alkyl, C1-C10-alkoxy, C1-C10-alkylthio, C1-C10-silyloxy, C1-C10-alkylamino, or electron-withdrawing groups selected from halogen atoms, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide.
16. The olefin metathesis catalyst of item 1 to 15, wherein R11, R12, R13 and R14 are independently hydrogen, unsubstituted C1-C12 alkyl, substituted C1-C12 alkyl, unsubstituted C4-C12 cycloalkyl, substituted C4-C12 cycloalkyl, unsubstituted C5-C24 aryl, substituted C5-C24 aryl, unsubstituted C5-C24 heteroaryl, substituted C5-C24 heteroaryl, unsubstituted C6-C24 aralkyl, substituted C6-C24 aralkyl, unsubstituted C6-C24 heteroaralkyl or substituted C6-C24 heteroaralkyl.
17. The olefin metathesis catalyst of item 1 to 16, wherein R2 is selected from hydrogen, straight chain or branched alkyl groups C1-C6-alkyl, C1-C6-alkoxy, C1-C6-alkylamino, C1-C6-dialkylamino, or electron-withdrawing groups selected from halogen atoms, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide.
18. The olefin metathesis catalyst of item 1 to 17, wherein R2 is selected from hydrogen, C1-C6-alkylamino, C1-C6-dialkylamino, or electron-withdrawing groups selected from F, Cl, Br and I, trifluormethyl (-CF₃), nitro (-NO₂), formyl (-CHO) or nitrile (-CN), in particular hydrogen, dimethylamino, diethylamino, dipropylamino, diisopropylamino, di-tert.-butylamino, nitro, chlorine and nitrile.
19. The olefin metathesis catalyst of item 1 to 18, wherein X1 and X2 are independently selected from Cl or Br, or X1 and X2 are oxygen or sulfur linked together by a hydrocarbon bridge being part of an aromatic ring or ring system forming a structure of formula 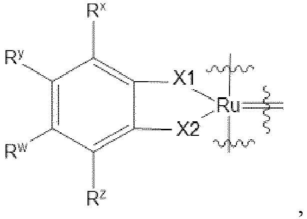 wherein R^{x}, R^{y}, R^{w} and R^{z} are independently independently hydrogen, halogen, unsubstituted C1-C10 alkyl, substituted C1-C10 alkyl, unsubstituted C3-C10 cycloalkyl, substituted C3-C10 cycloalkyl, unsubstituted C5-C24 aryl or substituted C5-C24 aryl, or at least two of R^{x}, R^{y}, R^{w} and R^{z} are linked together to form a mono- or polycyclic substituted or unsubstituted C3-C10 cycloalkyl or C5-C24 aryl ring or ring system; typically R^{x}, R^{y}, R^{w} and R^{z} are independently C1-C6 alkyl, hydrogen, unsubstituted phenyl, substituted phenyl, unsubstituted naphthyl, substituted naphthyl; or halogen; or R^{x} is hydrogen, methyl,tert.-butyl, phenyl or Cl, R^{y} and R^{w} are hydrogen or R^{y} and R^{w} are linked together to form a mono- or polycyclic C5-C24 aryl ring or ring system, R^{z} is hydrogen, methyl,tert.-butyl, phenyl or Cl.
20. The olefin metathesis catalyst of item 1 to 19, wherein R3 is 1-adamantyl, 2-adamantyl, or a structure of the formula 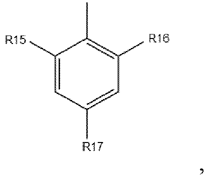 wherein R15, R16 and R17, independently of each other, are hydrogen, C1-C6-alkyl, C6-C10-aryl or a C1-C6-alkyl that is substituted with C6-C10-aryl.
21. The olefin metathesis catalyst of item 1 to 20, wherein R3 is 1-adamantyl, 2-adamantyl, unsubstituted phenyl, 2,4,6-trimethylphenyl, 2,6-di-*iso*-propylphenyl, 2-methyl-6-*tert*-butylphenyl, 2-*iso*-propyl-6-methylphenyl, 2-*iso*-propyl-phenyl, 2,6-di-ethylphenyl, 2-ethyl-6-methylphenyl or 2-methyl-phenyl.
22. The olefin metathesis catalyst of item 1 to 21, wherein R3a is C1-C6-alkyl, C6-C10-aryl or together with R4 can form a five, six or seven membered cycloalkyl or heterocyclic ring system with the carbon atom to which they are attached, or R3a is methyl, ethyl, *n-*propyl, isopropyl, n-butyl, sec-butyl, tert.-butyl or phenyl; or together with R4 can form a cyclopentyl, cyclohexyl or cycloheptyl ring, with the carbon atom to which they are attached.
23. The olefin metathesis catalyst of item 1 to 22, wherein R4 is C1-C6-alkyl, C6-C10-aryl or together with R3a can form a five, six or seven membered cycloalkyl or heterocyclic ring system with the carbon atom to which they are attached, or R4 is methyl, ethyl, *n*-propyl, isopropyl, n-butyl, sec-butyl, tert.-butyl or phenyl; or together with R3a can form a cyclopentyl, cyclohexyl or cycloheptyl ring, with the carbon atom to which they are attached.
24. The olefin metathesis catalyst of item 1 to 23, wherein R5, R6, R7 and R8 are, independently from each other, selected from hydrogen, straight chain or branched C1-C6-alkyl, C1-C6-alkoxy, C1-C6-alkylthio, C1-C6-silyloxy, C1-C6-alkylamino, or electron-withdrawing groups selected from F, Cl, Br and I, trifluormethyl (-CF₃), nitro (-NO₂), sulfinyl (-SO-), sulfonyl (-SO₂-), formyl (-CHO), C1-C6-carbonyl, C1-C6-carboxyl, C1-C6-alkylamido, C1-C6-aminocarbonyl, nitrile (-CN) or C1-C6-sulfonamide.
25. The olefin metathesis catalyst of item 1 to 24, wherein R11, R12, R13 and R14 are independently hydrogen, unsubstituted C1-C6 alkyl, substituted C1-C6 alkyl, unsubstituted C4-C8 cycloalkyl, substituted C4-C8 cycloalkyl, unsubstituted C5-C14 aryl, substituted C5-C1 aryl, unsubstituted C5-C14 heteroaryl, substituted C5-C1 heteroaryl, unsubstituted C6-C14 aralkyl, substituted C6-C1 aralkyl, unsubstituted C6-C14 heteroaralkyl or substituted C6-C14 heteroaralkyl.
26. The olefin metathesis catalyst of item 1 to 25, wherein R2 is selected from hydrogen, methyl, ethyl, propyl, isopropyl, n-butyl, isobutyl, tert.-butyl, methoxy, ethoxy, propoxy, isopropoxy, butoxy, isobutoxy, tert.-butoxy, methylamino, dimethylamino, ethylamino, dimethylamino, propylamino, dipropylamino, isopropylamino, diisopropylamino, butylamino, dibutylamino, isobutylamino, diisobutylamino, tert.-butylamino, di-tert.-butylamino, phenyl, naphthyl, phenoxy, F, Cl, Br, I, trifluormethyl (-CF₃), nitro (-NO₂), formyl (-CHO) or nitrile (-CN).
27. The olefin metathesis catalyst of item 1 to 26, wherein X1 and X2 are independently selected from Cl or Br, or X1 and X2 are sulfur linked together by a hydrocarbon bridge being part of an aromatic ring or ring system forming a structure of formula 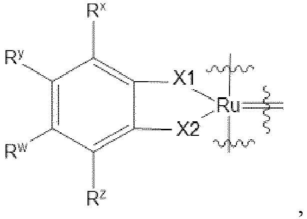 wherein R^{x}, R^{y}, R^{w} and R^{z} are independently hydrogen, methyl, ethyl, isopropyl, tert.-butyl, Br or Cl; or
   R^{x} is hydrogen, methyl, tert.-butyl, phenyl or Cl, R^{y} and R^{w} are hydrogen or, R^{z} is hydrogen, methyl, tert.-butyl, phenyl or Cl.
28. The olefin metathesis catalyst of item 1 to 27, wherein wherein R3 is 1-adamantyl, 2-adamantyl, or a structure of the formula 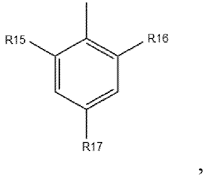 wherein R15, R16 and R17, independently of each other, are hydrogen, methyl, ethyl, propyl, isopropyl, n-butyl, isobutyl, tert. -butyl.
29. The olefin metathesis catalyst of item 1 to 28, wherein R3a is methyl, ethyl, *n*-propyl, isopropyl, n-butyl, iso-butyl, tert.-butyl or phenyl; or together with R4 can form a cyclopentyl, cyclohexyl or cycloheptyl ring, with the carbon atom to which they are attached
30. The olefin metathesis catalyst of item 1 to 29, wherein R4 is methyl, ethyl, *n*-propyl, isopropyl, n-butyl, sec-butyl, tert.-butyl or phenyl; or together with R3a can form a cyclopentyl, cyclohexyl or cycloheptyl ring, with the carbon atom to which they are attached.
31. The olefin metathesis catalyst of item 1 to 30, wherein R5, R6, R7 and R8 are, independently from each other, selected from hydrogen, hydrogen, methyl, ethyl, propyl, isopropyl, n-butyl, isobutyl, tert.-butyl, methoxy, ethoxy, propoxy, isopropoxy, butoxy, isobutoxy, tert.-butoxy, methylamino, dimethylamino, ethylamino, dimethylamino, propylamino, dipropylamino, isopropylamino, diisopropylamino, butylamino, dibutylamino, isobutylamino, diisobutylamino, tert.-butylamino, di-tert.-butylamino, or electron-withdrawing groups selected from F, Cl, Br and I, trifluormethyl (-CF₃), nitro (-NO₂), formyl (-CHO), C₁-C₆-carboxyl or nitrile (-CN); or wherein R5, R7 and R8 are, independently from each other, selected from hydrogen, hydrogen, methyl, ethyl, propyl, isopropyl, n-butyl, isobutyl, tert.-butyl and R6 is an electron-withdrawing groups selected from F, Cl, Br and I, trifluormethyl (-CF₃), nitro (-NO₂), formyl (-CHO), C1-C6-carboxyl or nitrile (-CN).
32. The olefin metathesis catalyst of item 1 to 31, wherein R11, R12, R13 and R14 are independently hydrogen, methyl, ethyl, propyl, isopropyl, n-butyl, isobutyl, tert.-butyl; or wherein R11 and R12 are methyl and R13 and R14 are hydrogen; or wherein R11 and R12 are hydrogen and R13 and R14 are methyl.
33. The olefin metathesis catalyst of item 1 to 31, wherein: R1 is hydrogen; R2 is selected from hydrogen, straight chain or branched alkyl groups including C1-C10-alkyl, C1-C10-alkoxy, C1-C10-alkylthio, C1-C10-silyloxy, C1-C10-alkylamino, C6-C14-aryl, C6-C14-aryloxy, C6-C14-heterocyclic or electron-withdrawing groups selected from halogen atoms, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide;
   X1 and X2 are halogen (specifically Cl, Br, I or F) or are independently oxygen or sulfur linked together by a hydrocarbon bridge being part of an aromatic ring or ring system independently;
   R3 is 1-adamantyl, 2-adamantyl, unsubstituted phenyl or substituted phenyl and in particular is 2,4,6-trimethylphenyl, 2,6-di-*iso*-propylphenyl, 2-methyl-6-*tert*-butylphenyl, 2-*iso*-propyl-6-methylphenyl, 2-*iso*-propyl-phenyl, 2,6-di-ethylphenyl, 2-ethyl-6-methylphenyl or 2-methyl-phenyl;
   R3a is methyl, ethyl, *n*-propyl, or phenyl; or together with R4 can form a five to ten membered cycloalkyl or heterocyclic ring, with the carbon atom to which they are attached; and
   R4 is methyl, ethyl, *n*-propyl, or phenyl; or together with R3a can form a five- to ten-membered cycloalkyl or heterocyclic ring, with the carbon atom to which they are attached; R11, R12, R13 and R14 are independently hydrogen, unsubstituted C1-C12 alkyl, substituted C1-C12 alkyl, unsubstituted C4-C12 cycloalkyl, substituted C4-C12 cycloalkyl, unsubstituted C5-C24 aryl, substituted C5-C24 aryl, unsubstituted C5-C24 heteroaryl, substituted C5-C24 heteroaryl, unsubstituted C6-C24 aralkyl, substituted C6-C24 aralkyl, unsubstituted C6-C24 heteroaralkyl or substituted C6-C24 heteroaralkyl;
   R5, R6, R7 and R8 are, independently from each other, selected from hydrogen, straight chain or branched alkyl groups including C1-C 10-alkyl, C1-C 10-alkoxy, C1-C 10-alkylthio, C1-C10-silyloxy, C1-C10-alkylamino, or electron-withdrawing groups selected from F, Cl, Br and I, trifluormethyl (-CF₃), nitro (-NO₂), sulfinyl (-SO-), sulfonyl (-SO₂-), formyl (-CHO), C₁-C₁₀-carbonyl, C₁-C₁₀-carboxyl, C₁-C₁₀-alkylamido, C₁-C₁₀-aminocarbonyl, nitrile (-CN) or C₁-C₁₀-sulfonamide.

### Detailed Description of the Invention.

The invention relates to olefin metathesis catalysts represented by **Formula (I)** 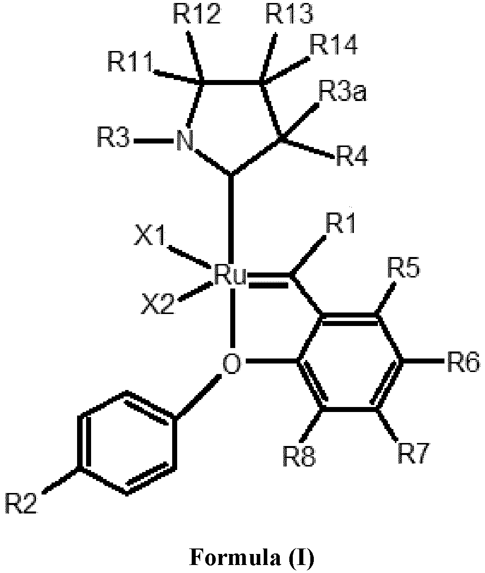 wherein:
R1 is hydrogen or alkyl, in particular hydrogen;
R2 is selected from hydrogen, straight chain alkyl, branched chain alkyl, cycloalkyl, alkoxy, alkylthio, silyloxy, alkylamino, dialkylamino, aryl, aryloxy, heterocyclic or an electron-withdrawing group;
X1 and X2 are the same or different anionic ligands and/or are linked together to form a ring or a ring system;
R3 is adamantyl, unsubstituted phenyl or substituted phenyl;
R3a is alkyl or aryl; or together with R4 can form a cycloalkyl or heterocyclic ring; and
R4 is alkyl or aryl; or together with R3a can form a cycloalkyl or heterocyclic ring;
R11, R12, R13 and R14 are independently hydrogen, unsubstituted alkyl, substituted alkyl, unsubstituted cycloalkyl, substituted cycloalkyl, unsubstituted aryl, substituted aryl, unsubstituted heteroaryl, substituted heteroaryl, unsubstituted aralkyl, substituted aralkyl, unsubstituted heteroaralkyl or substituted heteroaralkyl;
R5, R6, R7 and R8 are, independently from each other, selected from hydrogen, straight chain or branched alkyl groups, alkoxy, alkylthio, silyloxy, alkylamino, or electron-withdrawing groups.

In some instances, R1 is either hydrogen or selected from C1-C4-alkyl, more specifically from hydrogen, methyl, ethyl, propyl, isopropyl, n-butyl or tert.-butyl, but it has shown that usually R1 being hydrogen is sufficient.

In another embodiment, R2 is selected from hydrogen, C1-C30-alkyl, C1-C30-alkoxy, C1-C30-alkylthio, C1-C30-silyloxy, C1-C30-alkylamino, C1-C30-dialkylamino, C5-C30-aryl, CS-C30-aryloxy, C5-C30-heterocyclic or an electron-withdrawing group.

More specifically, R2 is selected from hydrogen, straight chain or branched alkyl groups including C1-C10-alkyl, C1-C10-alkoxy, C1-C10-alkylthio, C1-C10-silyloxy, C1-C10-alkylamino, C1-C10-dialkylamino, C6-C14-aryl, C6-C14-aryloxy, C6-C14-heterocyclic or an electron-withdrawing group; or
R2 is selected from hydrogen, straight chain or branched alkyl groups C1-C6-alkyl, C1-C6-alkoxy, C1-C6-alkylamino, C1-C6-dialkylamino, or electron-withdrawing groups selected from halogen atoms, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide, or
R2 is selected from hydrogen, C1-C6-alkylamino, C1-C6-dialkylamino, or electron-withdrawing groups selected from F, Cl, Br and I, trifluormethyl (-CF₃), nitro (-NO₂), formyl (-CHO) or nitrile (-CN), in particular hydrogen, dimethylamino, diethylamino, dipropylamino, diisopropylamino, di-tert.-butylamino, nitro, chlorine and nitrile.

Even more specifically, R2 is selected from hydrogen, methyl, ethyl, propyl, isopropyl, n-butyl, isobutyl, tert.-butyl, methoxy, ethoxy, propoxy, isopropoxy, butoxy, isobutoxy, tert.-butoxy, methylamino, dimethylamino, ethylamino, dimethylamino, propylamino, dipropylamino, isopropylamino, diisopropylamino, butylamino, dibutylamino, isobutylamino, diisobutylamino, tert.-butylamino, di-tert.-butylamino, phenyl, naphthyl, phenoxy, F, Cl, Br, I, trifluormethyl (-CF₃), nitro (-NO₂), formyl (-CHO) or nitrile (-CN).

In another embodiment, X1 and X2 are the same or different and halogen or X1 and X2 are a heteroatom and are connected by a C1-C5 bridge that may be saturated or unsaturated and can be part of an aromatic or aliphatic ring or ring system.

Specifically, X1 and X2 are independently selected from Cl, Br, I or F or are independently oxygen or sulfur linked together by a hydrocarbon bridge being part of an aromatic ring or ring system independently; or
X1 and X2 are independently selected from Cl, Br, I or F or are independently oxygen or sulfur linked together by a hydrocarbon bridge being part of an aromatic ring or ring system independently; or
X1 and X2 are independently selected from Cl or Br, or X1 and X2 are oxygen or sulfur linked together by a hydrocarbon bridge being part of an aromatic ring or ring system forming a structure of formula 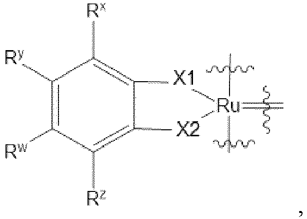 wherein R^{x}, R^{y}, R^{w} and R^{z} are independently independently hydrogen, halogen, unsubstituted C1-C10 alkyl, substituted C1-C10 alkyl, unsubstituted C3-C10 cycloalkyl, substituted C3-C10 cycloalkyl, unsubstituted C5-C24 aryl or substituted C5-C24 aryl, or at least two of R^{x}, R^{y}, R^{w} and R^{z} are linked together to form a mono- or polycyclic substituted or unsubstituted C3-C10 cycloalkyl or C5-C24 aryl ring or ring system; typically R^{x}, R^{y}, R^{w} and R^{z} are independently C1-C6 alkyl, hydrogen, unsubstituted phenyl, substituted phenyl, unsubstituted naphthyl, substituted naphthyl; or halogen; or R^{x} is hydrogen, methyl,tert.-butyl, phenyl or Cl, R^{y} and R^{w} are hydrogen or R^{y} and R^{w} are linked together to form a mono- or polycyclic C₅-C₂₄ aryl ring or ring system, R^{z} is hydrogen, methyl,tert.-butyl, phenyl or Cl.

More specifically, X¹ and X² are independently selected from Cl or Br, or X¹ and X² are sulfur linked together by a hydrocarbon bridge being part of an aromatic ring or ring system forming a structure of formula 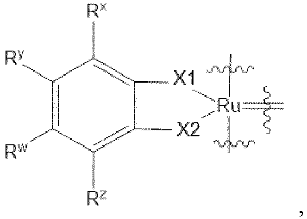 wherein R^{x}, R^{y}, R^{w} and R^{z} are independently hydrogen, methyl, ethyl, isopropyl,tert.-butyl, Br or Cl; or
R^{x} is hydrogen, methyl,tert.-butyl, phenyl or Cl, R^{y} and R^{w} are hydrogen or, R^{z} is hydrogen, methyl,tert.-butyl, phenyl or Cl.

In a further embodiment, R3 is adamantyl or a structure of the formula 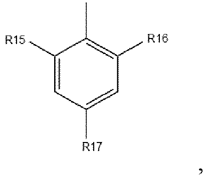 wherein R15, R16 and R17 are, independently of each other, are hydrogen, C1-C30-alkyl, C5-C30-aryl or a C1-C30-alkyl that is substituted with C5-C30-aryl; or
R3 is 1-adamantyl, 2-adamantyl or or a structure of the formula 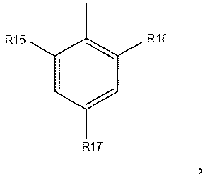 wherein R15, R16 and R17 are, independently of each other, are hydrogen, C1-C12-alkyl, C6-C14-aryl or a C1-C12-alkyl that is substituted with C6-C14-aryl; or R15, R16 and R17, independently of each other, are hydrogen, C1-C6-alkyl, C6-C10-aryl or a C1-C6-alkyl that is substituted with C6-C10-aryl; or wherein R3 is selected from the group consisting of 1-adamantyl, 2-adamantyl, unsubstituted phenyl, 2,4,6-trimethylphenyl, 2,6-di-*iso-*propylphenyl, 2-methyl-6-*tert*-butylphenyl, 2-*iso*-propyl-6-methylphenyl, 2-*iso*-propyl-phenyl, 2,6-di-ethylphenyl, 2-ethyl-6-methylphenyl or 2-methyl-phenyl.

In yet another embodiment, R3a is C1-C30-alkyl, C5-C30-aryl or together with R4 can form a four to fourteen membered cycloalkyl or heterocyclic ring system with the carbon atom to which they are attached; specifically, R3a is C1-C12-alkyl, C6-C14-aryl or together with R4 can form a five to ten membered cycloalkyl or heterocyclic ring system with the carbon atom to which they are attached; or
R3a is C1-C6-alkyl, C6-C10-aryl or together with R4 can form a five, six or seven membered cycloalkyl or heterocyclic ring system with the carbon atom to which they are attached, or
R3a is methyl, ethyl, *n*-propyl, isopropyl, n-butyl, sec-butyl, tert.-butyl or phenyl; or together with R4 can form a cyclopentyl, cyclohexyl or cycloheptyl ring, with the carbon atom to which they are attached.

In yet a further embodiment, R4 is C1-C30-alkyl, C5-C30-aryl or together with R3a can form a four to fourteen membered cycloalkyl or heterocyclic ring system with the carbon atom to which they are attached; or
R4 is C1-C12-alkyl, C6-C14-aryl or together with R3a can form a five to ten membered cycloalkyl or heterocyclic ring system with the carbon atom to which they are attached; or
R4 is C1-C6-alkyl, C6-C10-aryl or together with R3a can form a five, six or seven membered cycloalkyl or heterocyclic ring system with the carbon atom to which they are attached, or R4 is methyl, ethyl, *n*-propyl, isopropyl, n-butyl, sec-butyl, tert.-butyl or phenyl; or together with R3a can form a cyclopentyl, cyclohexyl or cycloheptyl ring, with the carbon atom to which they are attached.

A further embodiment relates to a metathesis catalyst wherein R5, R6, R7 and R8 are, independently from each other, selected from hydrogen, straight chain or branched C1-C30-alkyl, C1-C30-alkoxy, C1-C30-alkylthio, C1-C30-silyloxy, C1-C30-alkylamino, C1-C30-dialkylamino,or electron-withdrawing groups selected from halogen atoms, trifluormethyl (-CF₃), nitro (-NO₂), sulfinyl (-SO-), sulfonyl (-SO₂-), formyl (-CHO), C₁-C₃₀-carbonyl, C₁-C₃₀-carboxyl, C₁-C₃₀-alkylamido, C₁-C₃₀-aminocarbonyl, nitrile (-CN) or C₁-C₃₀-sulfonamide; or
R5, R6, R7 and R8 are, independently from each other, selected from hydrogen, straight chain or branched C1-C10-alkyl, C1-C10-alkoxy, C1-C10-alkylthio, C1-C10-silyloxy, C1-C10-alkylamino, or electron-withdrawing groups selected from halogen atoms, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide; or
R5, R6, R7 and R8 are, independently from each other, selected from hydrogen, straight chain or branched C1-C6-alkyl, C1-C6-alkoxy, C1-C6-alkylthio, C1-C6-silyloxy, C1-C6-alkylamino, or electron-withdrawing groups selected from F, Cl, Br and I, trifluormethyl (-CF₃), nitro (-NO₂), sulfinyl (-SO-), sulfonyl (-SO₂-), formyl (-CHO), C1-C6-carbonyl, C1-C6-carboxyl, C1-C6-alkylamido, C1-C6-aminocarbonyl, nitrile (-CN) or C1-C6-sulfonamide; or
R5, R6, R7 and R8 are, independently from each other, are selected from the group consisting of hydrogen, hydrogen, methyl, ethyl, propyl, isopropyl, n-butyl, isobutyl, tert.-butyl, methoxy, ethoxy, propoxy, isopropoxy, butoxy, isobutoxy, tert.-butoxy, methylamino, dimethylamino, ethylamino, dimethylamino, propylamino, dipropylamino, isopropylamino, diisopropylamino, butylamino, dibutylamino, isobutylamino, diisobutylamino, tert.-butylamino, di-tert.-butylamino, F, Cl, Br and I, trifluormethyl (-CF₃), nitro (-NO₂), formyl (-CHO), C₁-C₆-carboxyl or nitrile (-CN) and combinations thereof.

In an embodiment thereof, three of R5, R6, R7 and R8 are hydrogen or C1-C10-alkyl and one of R5, R6, R7 and R8 is selected from the definition of R5, R6, R7 and R8 as defined above, with the proviso that it is different from hydrogen; more specifically, R5, R7 and R8 are hydrogen or alkyl, in particular C1-C10-alkyl and R6 is an electron-withdrawing group as defined above; or
R5, R7 and R8 are, independently from each other, selected from hydrogen, methyl, ethyl, propyl, isopropyl, n-butyl, isobutyl, tert.-butyl and R6 is an electron-withdrawing groups selected from F, Cl, Br and I, trifluormethyl (-CF₃), nitro (-NO₂), formyl (-CHO), C1-C6-carboxyl or nitrile (-CN).

In yet another embodiment, R5, R6, R7 and R8 are hydrogen.

In another embodiment, R11, R12, R13 and R14 are independently hydrogen, unsubstituted C1-C30 alkyl, substituted C1-C30 alkyl, unsubstituted C4-C30 cycloalkyl, substituted C4-C30 cycloalkyl, unsubstituted C5-C30 aryl, substituted C5-C30 aryl, unsubstituted C5-C30 heteroaryl, substituted C5-C30 heteroaryl, unsubstituted C6-C30 aralkyl, substituted C6-C30 aralkyl, unsubstituted C6-C30 heteroaralkyl or substituted C6-C30 heteroaralkyl; or
R11, R12, R13 and R14 are independently hydrogen, unsubstituted C1-C12 alkyl, substituted C1-C12 alkyl, unsubstituted C4-C12 cycloalkyl, substituted C4-C12 cycloalkyl, unsubstituted C5-C24 aryl, substituted C5-C24 aryl, unsubstituted C5-C24 heteroaryl, substituted C5-C24 heteroaryl, unsubstituted C6-C24 aralkyl, substituted C6-C24 aralkyl, unsubstituted C6-C24 heteroaralkyl or substituted C6-C24 heteroaralkyl; or
R11, R12, R13 and R14 are independently hydrogen, unsubstituted C1-C6 alkyl, substituted C1-C6 alkyl, unsubstituted C4-C8 cycloalkyl, substituted C4-C8 cycloalkyl, unsubstituted C5-C14 aryl, substituted C5-C1 aryl, unsubstituted C5-C14 heteroaryl, substituted C5-C1 heteroaryl, unsubstituted C6-C14 aralkyl, substituted C6-C1 aralkyl, unsubstituted C6-C14 heteroaralkyl or substituted C6-C14 heteroaralkyl or, more specifically, wherein R11, R12, R13 and R14 are independently hydrogen, methyl, ethyl, propyl, isopropyl, n-butyl, isobutyl, tert.-butyl; or wherein R11 and R12 are methyl and R13 and R14 are hydrogen; or wherein R11 and R12 are hydrogen and R13 and R14 are methyl; or wherein R11, R12, R13 and R14 are hydrogen.

In a first specific embodiment, in the olefin metathesis catalyst of Formula (I) R1 is hydrogen; R² is selected from hydrogen, straight chain or branched alkyl groups including C1-C10-alkyl, C 1-C10-alkoxy, C1-C10-alkylthio, C1-C10-silyloxy, C 1-C10-alkylamino, C1-C10-dialkylamino, C6-C14-aryl, or an electron-withdrawing group;
X1 and X2 are independently selected from Cl, Br, I or F or are independently oxygen or sulfur linked together by a hydrocarbon bridge being part of an aromatic ring or ring system independently;
R3 is 1-adamantyl, 2-adamantyl or a structure of the formula 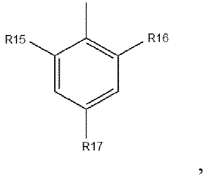 wherein
R15, R16 and R17 are, independently of each other, are hydrogen, C1-C12-alkyl;
R3a is C1-C12-alkyl, C6-C14-aryl.
R4 is C1-C12-alkyl.
R5, R6, R7 and R8 are, independently from each other, selected from hydrogen, straight chain or branched C1-C10-alkyl, or electron-withdrawing groups selected from halogen atoms, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide;
R11, R12, R13 and R14 are independently hydrogen, unsubstituted C1-C12 alkyl.

In a second specific embodiment, R1 is hydrogen; R2 is selected from hydrogen, straight chain or branched C1-C6-alkyl, C1-C6-dialkylamino, or electron-withdrawing groups selected from halogen atoms, trifluormethyl (-CF₃), nitro (-NO₂) or nitrile (-CN); even more specifically, R2 is selected from hydrogen, dimethylamino, diethylamino, dipropylamino, diisopropylamino, di-tert.-butylamino, nitro, chlorine and nitrile;
X¹ and X² are independently selected from Cl or Br, or X1 and X2 are oxygen or sulfur linked together by a hydrocarbon bridge being part of an aromatic ring or ring system forming a structure of formula 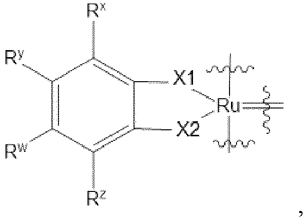 wherein R^{x}, R^{y}, R^{w} and R^{z} are independently independently hydrogen, halogen, unsubstituted C1-C10 alkyl, substituted C1-C10 alkyl, unsubstituted C3-C10 cycloalkyl, substituted C3-C10 cycloalkyl, unsubstituted C5-C24 aryl or substituted C5-C24 aryl, or at least two of R^{x}, R^{y}, R^{w} and R^{z} are linked together to form a mono- or polycyclic substituted or unsubstituted C3-C10 cycloalkyl or C5-C24 aryl ring or ring system; typically R^{x}, R^{y}, R^{w} and R^{z} are independently C1-C6 alkyl, hydrogen, unsubstituted phenyl, substituted phenyl, unsubstituted naphthyl, substituted naphthyl; or halogen; or R^{x} is hydrogen, methyl, tert.-butyl, phenyl or Cl, R^{y} and R^{w} are hydrogen or R^{y} and R^{w} are linked together to form a mono- or polycyclic C5-C24 aryl ring or ring system, R^{z} is hydrogen, methyl,tert.-butyl, phenyl or Cl.
R3 is 1-adamantyl, 2-adamantyl, or a structure of the formula 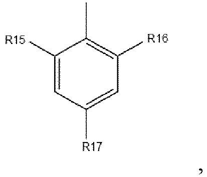 wherein R15, R16 and R17, independently of each other, are hydrogen, C1-C6-alkyl, C6-C10-aryl or a C1-C6-alkyl that is substituted with C6-C10-aryl.

More specifically, in thesceond embodiment it is also possible that R3 is 1-adamantyl, 2-adamantyl, unsubstituted phenyl, 2,4,6-trimethylphenyl, 2,6-di-*iso*-propylphenyl, 2-methyl-6-*tert*-butylphenyl, 2-*iso*-propyl-6-methylphenyl, 2-*iso*-propyl-phenyl, 2,6-di-ethylphenyl, 2-ethyl-6-methylphenyl or 2-methyl-phenyl;
R3a is C1-C6-alkyl, C6-C10-aryl or together with R4 can form a five, six or seven membered cycloalkyl or heterocyclic ring system with the carbon atom to which they are attached, or R3a is methyl, ethyl, *n*-propyl, isopropyl, n-butyl, sec-butyl, tert.-butyl or phenyl; or together with R4 can form a cyclopentyl, cyclohexyl or cycloheptyl ring, with the carbon atom to which they are attached; or
R4 is C1-C6-alkyl, C6-C10-aryl or together with R3a can form a five, six or seven membered cycloalkyl or heterocyclic ring system with the carbon atom to which they are attached, or R⁴ is methyl, ethyl, *n*-propyl, isopropyl, n-butyl, sec-butyl, tert.-butyl or phenyl; or together with R3a can form a cyclopentyl, cyclohexyl or cycloheptyl ring, with the carbon atom to which they are attached;
R5, R6, R7 and R8 are, independently from each other, selected from hydrogen, straight chain or branched C1-C6-alkyl, C1-C6-alkoxy, C1-C6-alkylthio, C1-C6-silyloxy, C1-C6-alkylamino, or electron-withdrawing groups selected from F, Cl, Br and I, trifluormethyl (-CF₃), nitro (-NO₂), sulfinyl (-SO-), sulfonyl (-SO₂-), formyl (-CHO), C1-C6-carbonyl, C1-C6-carboxyl, C1-C6-alkylamido, C1-C6-aminocarbonyl, nitrile (-CN) or C1-C6-sulfonamide; R11, R12, R13 and R14 are independently hydrogen, unsubstituted C1-C6 alkyl, substituted C1-C6 alkyl, unsubstituted C4-C8 cycloalkyl, substituted C4-C8 cycloalkyl, unsubstituted C5-C14 aryl, substituted C5-C1 aryl, unsubstituted C5-C14 heteroaryl, substituted C5-C1 heteroaryl, unsubstituted C6-C14 aralkyl, substituted C6-C1 aralkyl, unsubstituted C6-C14 heteroaralkyl or substituted C6-C14 heteroaralkyl.

In a third specific embodiment, R1 is hydrogen, R2 is selected from hydrogen, methyl, ethyl, propyl, isopropyl, n-butyl, isobutyl, tert.-butyl, methoxy, ethoxy, propoxy, isopropoxy, butoxy, isobutoxy, tert.-butoxy, methylamino, dimethylamino, ethylamino, dimethylamino, propylamino, dipropylamino, isopropylamino, diisopropylamino, butylamino, dibutylamino, isobutylamino, diisobutylamino, tert.-butylamino, di-tert.-butylamino, phenyl, naphthyl, phenoxy, F, Cl, Br, I, trifluormethyl (-CF₃), nitro (-NO₂), formyl (-CHO) or nitrile (-CN);
X1 and X2 are independently selected from Cl or Br, or X1 and X2 are sulfur linked together by a hydrocarbon bridge being part of an aromatic ring or ring system forming a structure of formula 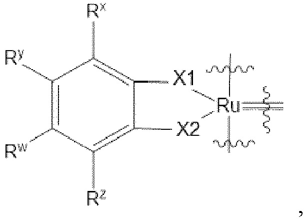 wherein R^{x}, R^{y}, R^{w} and R^{z} are independently hydrogen, methyl, ethyl, isopropyl,tert.-butyl, Br or Cl; or
R^{x} is hydrogen, methyl,tert.-butyl, phenyl or Cl, R^{y} and R^{w} are hydrogen or, R^{z} is hydrogen, methyl,tert.-butyl, phenyl or Cl;
R3 is 1-adamantyl, 2-adamantyl, or a structure of the formula 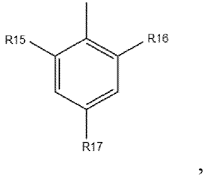
wherein R15, R16 and R17, independently of each other, are hydrogen, methyl, ethyl, propyl, isopropyl, n-butyl, isobutyl, tert.-butyl;
R3a is methyl, ethyl, *n*-propyl, isopropyl, n-butyl, iso-butyl, tert.-butyl or phenyl; or together with R⁴ can form a cyclopentyl, cyclohexyl or cycloheptyl ring, with the carbon atom to which they are attached;
R4 is methyl, ethyl, *n*-propyl, isopropyl, n-butyl, sec-butyl, tert.-butyl or phenyl; or together with R3a can form a cyclopentyl, cyclohexyl or cycloheptyl ring, with the carbon atom to which they are attached;
R5, R6, R7 and R8 are, independently from each other, selected from hydrogen, hydrogen, methyl, ethyl, propyl, isopropyl, n-butyl, isobutyl, tert.-butyl, methoxy, ethoxy, propoxy, isopropoxy, butoxy, isobutoxy, tert.-butoxy, methylamino, dimethylamino, ethylamino, dimethylamino, propylamino, dipropylamino, isopropylamino, diisopropylamino, butylamino, dibutylamino, isobutylamino, diisobutylamino, tert.-butylamino, di-tert.-butylamino, or electron-withdrawing groups selected from F, Cl, Br and I, trifluormethyl (-CF₃), nitro (-NO₂), formyl (-CHO), C1-C6-carboxyl or nitrile (-CN); or
wherein R5, R7 and R8 are, independently from each other, selected from hydrogen, hydrogen, methyl, ethyl, propyl, isopropyl, n-butyl, isobutyl, tert.-butyl and R6 is an electron-withdrawing groups selected from F, Cl, Br and I, trifluormethyl (-CF₃), nitro (-NO₂), formyl (-CHO), C1-C6-carboxyl or nitrile (-CN).
R11, R12, R13 and R14 are independently hydrogen, methyl, ethyl, propyl, isopropyl, n-butyl, isobutyl, tert.-butyl; or wherein R11 and R12 are methyl and R13 and R14 are hydrogen; or wherein R11 and R12 are hydrogen and R13 and R14 are methyl.

In a fourth specific embodiment, R1 is hydrogen;
R2 is selected from hydrogen, straight chain or branched alkyl groups including C1-C10-alkyl, C1-C10-alkoxy, C1-C10-alkylthio, C1-C10-silyloxy, C1-C10-alkylamino, C6-C14-aryl, C6-C14-aryloxy, C6-C14-heterocyclic or electron-withdrawing groups selected from halogen atoms, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide;
X1 and X2 are halogen (specifically Cl, Br, I or F) or are independently oxygen or sulfur linked together by a hydrocarbon bridge being part of an aromatic ring or ring system independently;
R3 is adamantyl, unsubstituted phenyl or substituted phenyl, 2,4,6-trimethylphenyl, 2,6-di-*iso*-propylphenyl, 2-methyl-6-*tert*-butylphenyl, 2-*iso*-propyl-6-methylphenyl, 2-*iso*-propyl-phenyl, 2,6-di-ethylphenyl, 2-ethyl-6-methylphenyl or 2-methyl-phenyl;
R3a is methyl, ethyl, *n*-propyl, or phenyl; or together with R4 can form a five to ten membered cycloalkyl or heterocyclic ring, with the carbon atom to which they are attached; R4 is methyl, ethyl, *n*-propyl, or phenyl; or together with R3a can form a five- to ten-membered cycloalkyl or heterocyclic ring, with the carbon atom to which they are attached; R11, R12, R13 and R14 are independently hydrogen, unsubstituted C1-C12 alkyl, substituted C1-C12 alkyl, unsubstituted C4-C12 cycloalkyl, substituted C4-C12 cycloalkyl, unsubstituted C5-C24 aryl, substituted C5-C24 aryl, unsubstituted C5-C24 heteroaryl, substituted C5-C24 heteroaryl, unsubstituted C6-C24 aralkyl, substituted C6-C24 aralkyl, unsubstituted C6-C24 heteroaralkyl or substituted C6-C24 heteroaralkyl;
R5, R6, R7 and R8 are, independently from each other, selected from hydrogen, straight chain or branched alkyl groups including C1-C10-alkyl, C1-C10-alkoxy, C1-C10-alkylthio, C 1-C10-silyloxy, C1-C10-alkylamino, or electron-withdrawing groups selected from F, Cl, Br and I, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide.

In a fifth specific embodiment, R1 is hydrogen;
R2 is selected from hydrogen, dimethylamino, diethylamino, dipropylamino, diisopropylamino, di-tert.-butylamino, nitro, chlorine and nitrile;
X1 and X2 are halogen, specifically Cl, Br, I or F, or are independently oxygen or sulfur linked together by a hydrocarbon bridge being part of an aromatic ring or ring system independently;
R3 is adamantyl, unsubstituted phenyl or substituted phenyl, 2,4,6-trimethylphenyl, 2,6-di-*iso*-propylphenyl, 2-methyl-6-*tert*-butylphenyl, 2-*iso*-propyl-6-methylphenyl, 2-*iso*-propyl-phenyl, 2,6-di-ethylphenyl, 2-ethyl-6-methylphenyl or 2-methyl-phenyl;
R3a is methyl, ethyl, *n*-propyl, or phenyl; or together with R4 can form a five to ten membered cycloalkyl, with the carbon atom to which they are attached;
R4 is methyl, ethyl, *n*-propyl, or phenyl; or together with R3a can form a five- to ten-membered cycloalkyl ring, with the carbon atom to which they are attached;
R11, R12, R13 and R14 are independently hydrogen, unsubstituted C1-C12 alkyl, substituted C1-C12 alkyl, unsubstituted C4-C12 cycloalkyl, substituted C4-C12 cycloalkyl, unsubstituted C5-C24 aryl, substituted C5-C24 aryl, unsubstituted C5-C24 heteroaryl, substituted C5-C24 heteroaryl, unsubstituted C6-C24 aralkyl, substituted C6-C24 aralkyl, unsubstituted C6-C24 heteroaralkyl or substituted C6-C24 heteroaralkyl;
R5, R6, R7 and R8 are, independently from each other, selected from hydrogen, straight chain or branched alkyl groups including C1-C10-alkyl, C 1-C 10-alkoxy, C1-C10-alkylthio, C 1-
C10-silyloxy, C1-C10-alkylamino, or electron-withdrawing groups selected from F, Cl, Br and I, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide.

In a sixth specific embodiment, R1 is hydrogen;
R2 is selected from hydrogen, dimethylamino, diethylamino, dipropylamino, diisopropylamino, di-tert.-butylamino, nitro, chlorine and nitrile;
X1 and X2 are Cl, Br, I, F or combinations thereof, or are independently oxygen or sulfur linked together by a hydrocarbon bridge being part of an aromatic ring or ring system independently;
R3 is 1-adamantyl, 2-adamantyl, unsubstituted phenyl or substituted phenyl, 2,4,6-trimethylphenyl, 2,6-di-*iso*-propylphenyl, 2-methyl-6-*tert*-butylphenyl, 2-*iso*-propyl-6-methylphenyl, 2-*iso*-propyl-phenyl, 2,6-di-ethylphenyl, 2-ethyl-6-methylphenyl or 2-methyl-phenyl;
R3a is methyl, ethyl, *n*-propyl, or phenyl;
R4 is methyl, ethyl, *n*-propyl, or phenyl;
R11, R12, R13 and R14 are independently hydrogen;
R5, R6, R7 and R8 are, independently from each other, selected from hydrogen or electron-withdrawing groups selected from F, Cl, Br and I, trifluormethyl (-CF₃), nitro (-NO₂), nitrile (-CN). More specifically, R5, R7 and R8 are hydrogen and R6 is selected from F, Cl, Br, I, trifluormethyl (-CF₃), nitro (-NO₂), nitrile (-CN).

The Invention is illustrated by the following examples.

### Examples:

### Conditions of GC measurements:

Volatile products were analyzed using an Agilent 6850 gas chromatography (GC) instrument with a flame ionization detector (FID). The following conditions and equipment were used:
Column: Agilent DB-5, 30 m x 0.25 mm (ID) x 0.25 µm film thickness.
Inlet temperature: 300 °C
Split ratio 20:1
Split flow: 20 ml/min
Total flow: 23.5 ml/min
Gas type: Helium
Oven: Starting temperature: 50 °C, hold time: 0.5 minutes.
   Ramp 1 rate 15 °C/min to 150 °C, final time: 0 minutes.
   Ramp 2 rate 30 °C/min to 320 °C, final time: 2.5 minutes
   Detector temperature: 325 °C

### Preparation of catalysts

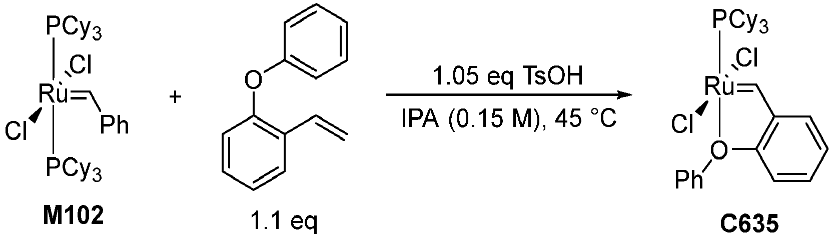

Synthesis of C635 (648-70): To a 250 mL RBF equipped with a magnetic stir bar was added 1-phenoxy-2-vinylbenzene (5.25 g, 26.7 mmol, 1.1 eq), M102 (20.0 g, 24.3 mmol, 1.0 eq), followed by isopropanol (165 mL). The mixture was stirred at 45 °C. After 2 h complete conversion of M102 was verified by NMR spectroscopy. The mixture was subsequently cooled in the freezer for 1 h, the brown solid was isolated by filtration, washed MeOH, IPA and hexanes, then dried under vacuum to yield C635 (14.3 g, 92.5% yield, 98.8+/-1.8% purity by NMR (IS 1,3,5-trimethoxybenzene), corrected yield for purity by ¹H-NMR 91.4%).
¹H NMR (400 MHz, CD₂Cl₂) δ 17.41 (d, *J* = 4.5 Hz, 1H), 7.88-7.71 (m, 1H), 7.64-7.47 (m, 5H), 7.47-7.38 (m, 1H), 7.21 (t, *J* = 7.5 Hz, 1H), 6.76 (d, *J* = 8.4 Hz, 1H), 2.33 (q, *J =* 12.1 Hz, 3H), 2.07 (m, 6H), 1.96-1.59 (m, 16H), 1.29 (br s, 9H).
³¹P NMR (162 MHz, CD₂Cl₂) δ 62.77 (s)

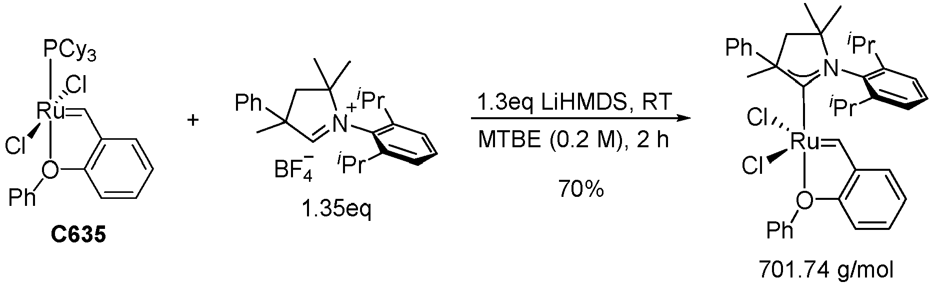

**Synthesis of C702:** To a 40 mL septa vial equipped with a magnetic stir bar was added ligand (0.463 g, 1.06 mmol, 1.35 eq), LiHMDS (97%, 0.177 g, 1.024 mmol, 1.30 eq), C635 (0.500 g, 0.788 mmol, 1.00 eq), and MTBE (4 mL). The mixture was stirred at 60 °C for 2 h and complete conversion of C635 was confirmed by NMR spectroscopy. The green solid was isolated by filtration, washed with MeOH and dried under vacuum to yield C702 (0.400 g, 72.3% yield in 98.3% purity by NMR (IS anthracene), corrected yield for purity by ¹H-NMR 71.1%).

¹H-NMR (CD₂Cl₂, 400MHz): δ 16.8 (s, 1H), 8.4 (d, *J* = 7.2 Hz, 2H), 7.8-7.4 (m, 9H), 7.2-6.8 (m, 5H), 6.7 (d, *J* = 8.2 Hz, 1H), 3.2 (m, 2H), 2.9 (m, 1H), 2.3 (d, *J* = 11.8 Hz, 1H), 2.2 (s, 3H), 2.8 (s, 3H), 2.6-2.2 (s, 9H), 0.9 (d, *J* = 5.9 Hz, 3H), 0.6 (d, *J* = 5.9 Hz, 3H).

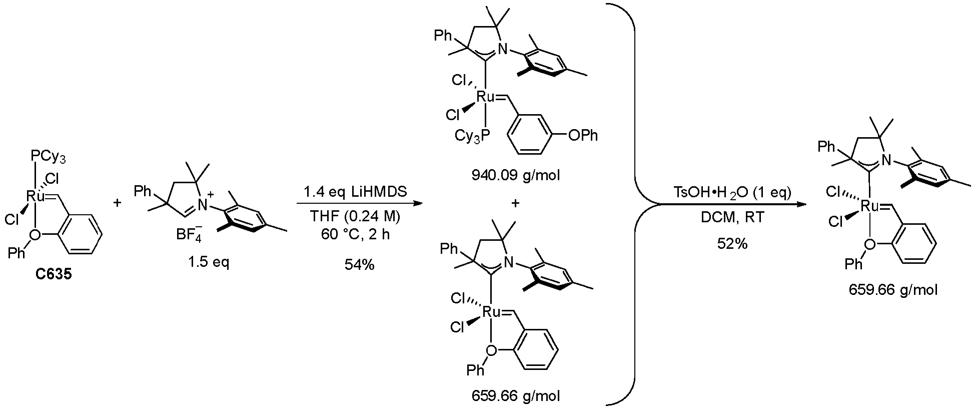

**Synthesis of C660:** To a 20 mL septa vial equipped with a magnetic stir bar were added ligand (0.930 g, 2.36 mmol, 1.5 eq), LiHMDS (97%, 0.381 g, 2.21 mmol, 1.4 eq), C635 (1.000 g, 1.58 mmol, 1.0 eq), and MTBE (6.5 mL). The brown mixture was stirred at 60 °C and changed color from brown to green. After 1.5 hours complete conversion of C635 was observed by NMR spectroscopy. All volatiles were subsequently removed, and the residue was triturated with hexanes for 1 h. The resulting brown solid was isolated by filtration, washed with hexane and MeOH then dried under vacuum to yield C940 (0.794 g, 53.5% yield. The mixed material was used in the next step without further purification.

To a 20 mL septa vial equipped with a magnetic stir bar was added TsOH•H₂O (0.139 g, 0.730 mmol, 1.0 eq), the solid from the previous reaction (0.686 mg, 0.730 mmol, 1.0 eq), and dichloromethane (3 mL). The mixture was stirred at 30 °C for 0.5 h then filtered through a silica gel plug. All volatiles were removed, and hexanes were added resulting in an oily green precipitate. The vial was placed in the freezer for 3 h before the green solid was isolated by filtration and washed with hexane. MeOH was added to the solid and the mixture stored in the freezer overnight. The resulting green solid was isolated by filtration, washed with MeOH and hexane, then dried under vacuum to yield C660 (0.170 g, 95.8% purity by NMR (IS anthracene), corrected yield 33.8%).

¹H-NMR (CD₂Cl₂, 400MHz): δ 16.5 (br s, 1H), 8.4 (br s, 2H), 7.8-7.0 (m, 13H), 6.7 (d, *J* = 8.2 Hz, 1H), 3.1 (m, 1H), 2.6-2.3 (m, 7H), 2.2-2.0 (m, 6H), 1.7-1.3 (m, 6H).

### Examples for Catalytic Activity

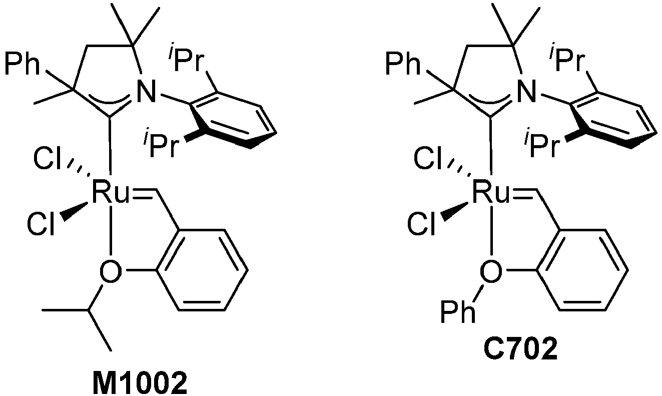

Metathesis reactions were carried out with C702 as Example and M1002 as Comparative Example.

### Self-metathesis of methyl dec-9-enoate (Me9DA) with 10 ppm catalyst at 35 °C

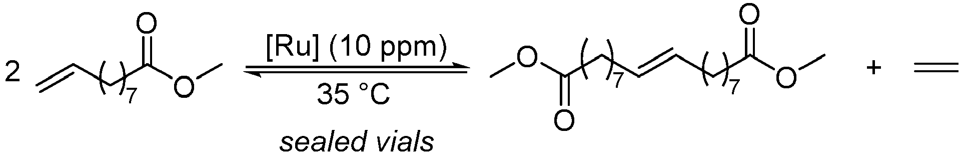

### Abbreviations:

Me9DA = methyl dec-9-enoate
Me9DA iso = isomers of methyl dec-9-enoate
DE = dimethyloctadec-9-enedioate (product)
GC: gas chromatography

### General procedure for self-metathesis of Me9DA to dimethyloctadec-9-enedioate (DE)

Prior to use, Me9DA was distilled under vacuum from potassium carbonate and stored in an argon filled glove box.

Me9DA (300 mg, 1.63 mmol) was weighed into a 40 mL septa vial equipped with a magnetic stir bar. Catalyst (10 ppm) was added as a stock solution in toluene. Vials were sealed and placed on a preheated aluminum block (35 °C) with stirring. Reaction aliquots were acquired at the specified times and were treated with 1.0 M trishydroxymethyl phosphine solution in IPA for 15 min at 70 °C. Mixtures were subsequently partitioned between water and ethyl acetate (1:2), the organic layer extracted, and analyzed by GC.

The reaction carried out with C702 is an example, with M1002 as catalyst is a comparative example.

| | | | GC (%) | | | | |
|---|---|---|---|---|---|---|---|
| Catalyst [Ru] | Temp/°C | Time/h | Conversion | Me9DA | Me9DA iso | Other | DE |
| M1002 | 35 | 1 | 9 | 91 | 0.9 | 1.2 | 6.8 |
| | | 3 | 47 | 53 | 1.0 | 0.8 | 45.2 |
| | | 6 | 67 | 33 | 1.1 | 1.9 | 64 |
| C702 | 35 | 1 | 49 | 51 | 1 | 1.6 | 46.7 |
| | | 3 | 70 | 30 | 1,3 | 1.6 | 67.4 |
| | | 6 | 78 | 22 | 1.7 | 1.5 | 75 |

It can be seen that C702 afforded higher conversions of Me9DA and higher yields of dimethyloctadec-9-enedioate at a shorter reaction time when compared to M1002.

### Self-metathesis of 1-decene with 10 ppm catalyst at 35 °C

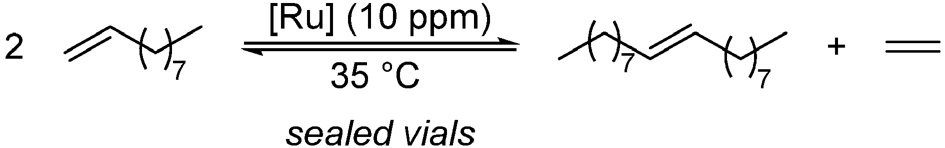

### Abbreviations:

1C10 = 1-decene
1C10 iso = isomers of 1-decene
9C18 = octadec-9-ene (product)
GC: gas chromatography

### General procedure for self-metathesis of 1-decene

1-decene was stored in the glove box over super activated alumina and filtered through a syringe filter prior to use.

1-decene (0.300 g, 2.14 mmol) was weighed into a 40 mL septa vial equipped with a magnetic stir bar. Catalyst (10 ppm) was added as a stock solution in toluene. The vial was sealed and placed on a preheated aluminum block (35 °C) with stirring. Reaction aliquots were acquired at the specified times and were treated with 1.0 M trishydroxymethyl phosphine solution in IPA for 15 min at 70 °C. Mixtures were subsequently partitioned between water and ethyl acetate (1:2), the organic layer extracted, and analyzed by GC.

| | | | GC (%) | | | | |
|---|---|---|---|---|---|---|---|
| Catalyst [Ru] | Temp/°C | Time/h | Conversion | 1C10 | 1C10 iso | Other | 9C18 |
| M1002 | 35 | 1 | 39 | 61 | 2 | 3 | 35 |
| | | 3 | 64 | 36 | 2 | 3 | 59 |
| | | 6 | 76 | 24 | 2 | 3 | 70 |
| | | 24 | 84 | 16 | 3 | 5 | 77 |
| C702 | 35 | 1 | 55 | 45 | 0 | 3 | 51 |
| | | 3 | 75 | 25 | 1 | 3 | 70 |
| | | 6 | 81 | 19 | 2 | 4 | 76 |
| | | 24 | 87 | 13 | 4 | 5 | 79 |

C702 afforded higher conversion of 1-decene and higher yields of octadec-9-ene at earlier timepoints compared to M1002.

## Claims

1. An olefin metathesis catalyst represented by the structure of **Formula (I)** 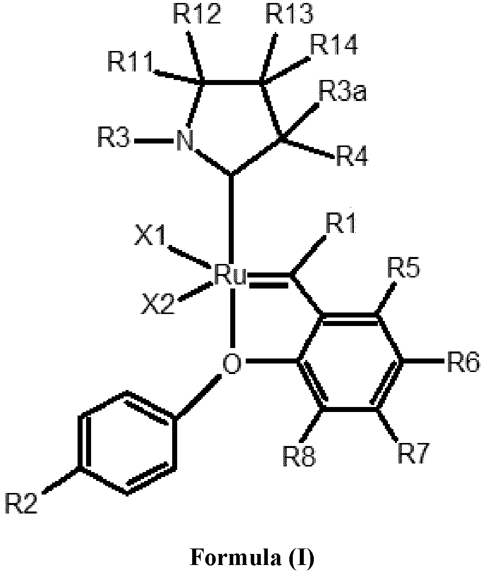 wherein:
R1 is hydrogen or alkyl;
R2 is selected from hydrogen, straight chain alkyl, branched chain alkyl, cycloalkyl, alkoxy, alkylthio, silyloxy, alkylamino, dialkylamino, aryl, aryloxy, heterocyclic or an electron-withdrawing group;
X1 and X2 are the same or different anionic ligands and/or are linked together to form a ring or a ring system;
R3 is adamantyl, unsubstituted phenyl or substituted phenyl;
R3a is alkyl or aryl; or together with R4 can form a cycloalkyl or heterocyclic ring; and
R4 is alkyl or aryl; or together with R3a can form a cycloalkyl or heterocyclic ring;
R11, R12, R13 and R14 are independently hydrogen, unsubstituted alkyl, substituted alkyl, unsubstituted cycloalkyl, substituted cycloalkyl, unsubstituted aryl, substituted aryl, unsubstituted heteroaryl, substituted heteroaryl, unsubstituted aralkyl, substituted aralkyl, unsubstituted heteroaralkyl or substituted heteroaralkyl;
R5, R6, R7 and R8 are, independently from each other, selected from hydrogen, straight chain or branched alkyl groups, alkoxy, alkylthio, silyloxy, alkylamino, or electron-withdrawing groups.

2. The olefin metathesis catalyst of claim 1, wherein R1 is hydrogen.

3. The olefin metathesis catalyst of claim 1 to 2, wherein R2 is selected from hydrogen, C1-C30-alkyl, C1-C30-alkoxy, C1-C30-alkylthio, C1-C30-silyloxy, C1-C30-alkylamino, C1-C30-dialkylamino, C5-C30-aryl, CS-C30-aryloxy, C5-C30-heterocyclic or an electron-withdrawing group.

4. The olefin metathesis catalyst of claim 1 to 3, wherein X1 and X2 are the same or different and halogen or X1 and X2 are a heteroatom and are connected by a C1-C5 bridge that may be saturated or unsaturated and can be part of an aromatic or aliphatic ring or ring system.

5. The olefin metathesis catalyst of claim 1 to 4, wherein R3 is adamantyl or a structure of the formula 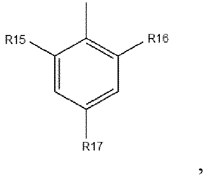 wherein R15, R16 and R17 are, independently of each other, are hydrogen, C1-C30-alkyl, C5-C30-aryl or a C1-C30-alkyl that is substituted with C5-C30-aryl.

6. The olefin metathesis catalyst of claim 1 to 5, wherein R3a is C1-C30-alkyl, C5-C30-aryl or together with R4 can form a four to fourteen membered cycloalkyl or heterocyclic ring system with the carbon atom to which they are attached.

7. The olefin metathesis catalyst of claim 1 to 6, wherein R4 is C1-C30-alkyl, C5-C30-aryl or together with R3a can form a four to fourteen membered cycloalkyl or heterocyclic ring system with the carbon atom to which they are attached.

8. The olefin metathesis catalyst of claim 1 to 7, wherein R5, R6, R7 and R8 are, independently from each other, selected from hydrogen, straight chain or branched C1-C30-alkyl, C1-C30-alkoxy, C1-C30-alkylthio, C1-C30-silyloxy, C1-C30-alkylamino, or electron-withdrawing groups selected from halogen atoms, trifluormethyl (-CF₃), nitro (-NO₂), sulfinyl (-SO-), sulfonyl (-SO₂-), formyl (-CHO), C1-C30-carbonyl, C1-C30-carboxyl, C1-C30-alkylamido, C1-C30-aminocarbonyl, nitrile (-CN) or C1-C30-sulfonamide.

9. The olefin metathesis catalyst of claim 1 to 8, wherein R11, R12, R13 and R14 are independently hydrogen, unsubstituted C1-C30 alkyl, substituted C1-C30 alkyl, unsubstituted C4-C30 cycloalkyl, substituted C4-C30 cycloalkyl, unsubstituted C5-C30 aryl, substituted C5-C30 aryl, unsubstituted C5-C30 heteroaryl, substituted C5-C30 heteroaryl, unsubstituted C6-C30 aralkyl, substituted C6-C30 aralkyl, unsubstituted C6-C30 heteroaralkyl or substituted C6-C30 heteroaralkyl.

10. The olefin metathesis catalyst of claim 1 to 9, wherein R2 is selected from hydrogen, straight chain or branched alkyl groups including C1-C10-alkyl, C1-C10-alkoxy, C 1-C10-alkylthio, C1-C10-silyloxy, C1-C10-alkylamino, C1-C10-dialkylamino, C6-C14-aryl, C6-C14-aryloxy, C6-C14-heterocyclic or an electron-withdrawing group.

11. The olefin metathesis catalyst of claim 1 to 10, wherein X1 and X2 are independently selected from Cl, Br, I or F or are independently oxygen or sulfur linked together by a hydrocarbon bridge being part of an aromatic ring or ring system independently.

12. The olefin metathesis catalyst of claim 1 to 11, wherein R3 is 1-adamantyl, 2-adamantyl or a structure of the formula 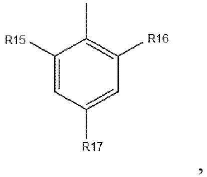 wherein R15, R16 and R17 are, independently of each other, are hydrogen, C1-C12-alkyl, C6-C14-aryl or a C1-C12-alkyl that is substituted with C6-C14-aryl.

13. The olefin metathesis catalyst of claim 1 to 12, wherein R2 is selected from hydrogen, straight chain or branched alkyl groups C1-C6-alkyl, C1-C6-alkoxy, C1-C6-alkylamino, C1-C6-dialkylamino, or electron-withdrawing groups selected from halogen atoms, trifluormethyl (-CF₃), nitro (-NO₂), sulfinyl (-SO-), sulfonyl (-SO₂-), formyl (-CHO), C1-C10-carbonyl, C1-C10-carboxyl, C1-C10-alkylamido, C1-C10-aminocarbonyl, nitrile (-CN) or C1-C10-sulfonamide.

14. The olefin metathesis catalyst of claim 1 to 13, wherein R2 is selected from hydrogen, C1-C6-alkylamino, C1-C6-dialkylamino, or electron-withdrawing groups selected from F, Cl, Br and I, trifluormethyl (-CF₃), nitro (-NO₂), formyl (-CHO) or nitrile (-CN), in particular hydrogen, dimethylamino, diethylamino, dipropylamino, diisopropylamino, di-tert.-butylamino, nitro, chlorine and nitrile.

15. The olefin metathesis catalyst of claim 1 to 14, wherein X1 and X2 are independently selected from Cl or Br, or X1 and X2 are oxygen or sulfur linked together by a hydrocarbon bridge being part of an aromatic ring or ring system forming a structure of formula 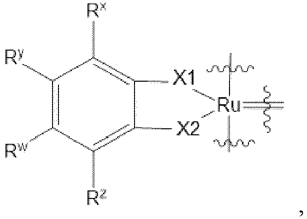 wherein R^{x}, R^{y}, R^{w} and R^{z} are independently independently hydrogen, halogen, unsubstituted C1-C10 alkyl, substituted C1-C10 alkyl, unsubstituted C3-C10 cycloalkyl, substituted C3-C10 cycloalkyl, unsubstituted C5-C24 aryl or substituted C5-C24 aryl, or at least two of R^{x}, R^{y}, R^{w} and R^{z} are linked together to form a mono- or polycyclic substituted or unsubstituted C3-C10 cycloalkyl or C5-C24 aryl ring or ring system; typically R^{x}, R^{y}, R^{w} and R^{z} are independently C1-C6 alkyl, hydrogen, unsubstituted phenyl, substituted phenyl, unsubstituted naphthyl, substituted naphthyl; or halogen; or R^{x} is hydrogen, methyl,tert.-butyl, phenyl or Cl, R^{y} and R^{w} are hydrogen or R^{y} and R^{w} are linked together to form a mono- or polycyclic C5-C24 aryl ring or ring system, R^{z} is hydrogen, methyl,tert.-butyl, phenyl or Cl.

16. The olefin metathesis catalyst of claim 1 to 15, wherein R³ is 1-adamantyl, 2-adamantyl, or a structure of the formula 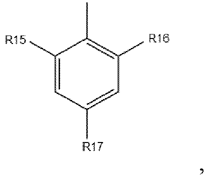 wherein R15, R16 and R17, independently of each other, are hydrogen, C1-C6-alkyl, C6-C10-aryl or a C1-C6-alkyl that is substituted with C6-C10-aryl.

17. The olefin metathesis catalyst of claim 1 to 16, wherein R2 is selected from hydrogen, methyl, ethyl, propyl, isopropyl, n-butyl, isobutyl, tert.-butyl, methoxy, ethoxy, propoxy, isopropoxy, butoxy, isobutoxy, tert.-butoxy, methylamino, dimethylamino, ethylamino, dimethylamino, propylamino, dipropylamino, isopropylamino, diisopropylamino, butylamino, dibutylamino, isobutylamino, diisobutylamino, tert.-butylamino, di-tert.-butylamino, phenyl, naphthyl, phenoxy, F, Cl, Br, I, trifluormethyl (-CF₃), nitro (-NO₂), formyl (-CHO) or nitrile (-CN).

18. The olefin metathesis catalyst of claim 1 to 17, wherein X1 and X2 are independently selected from Cl or Br, or X1 and X2 are sulfur linked together by a hydrocarbon bridge being part of an aromatic ring or ring system forming a structure of formula 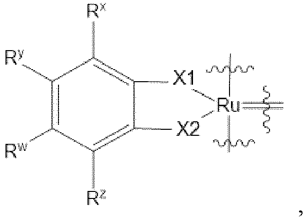 wherein R^{x}, R^{y}, R^{w} and R^{z} are independently hydrogen, methyl, ethyl, isopropyl,tert.-butyl, Br or Cl; or
R^{x} is hydrogen, methyl,tert.-butyl, phenyl or Cl, R^{y} and R^{w} are hydrogen or, R^{z} is hydrogen, methyl,tert.-butyl, phenyl or Cl.

19. The olefin metathesis catalyst of claim 1 to 18, wherein wherein R3 is 1-adamantyl, 2-adamantyl, or a structure of the formula 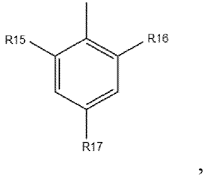 wherein R15, R16 and R17, independently of each other, are hydrogen, methyl, ethyl, propyl, isopropyl, n-butyl, isobutyl, tert.-butyl.

20. The olefin metathesis catalyst of claim 1 to 19, wherein R^{3a} is methyl, ethyl, n-propyl, isopropyl, n-butyl, iso-butyl, tert.-butyl or phenyl; or together with R⁴ can form a cyclopentyl, cyclohexyl or cycloheptyl ring, with the carbon atom to which they are attached.

21. The olefin metathesis catalyst of claim 1 to 20, wherein R4 is methyl, ethyl, n-propyl, isopropyl, n-butyl, sec-butyl, tert.-butyl or phenyl; or together with R3a can form a cyclopentyl, cyclohexyl or cycloheptyl ring, with the carbon atom to which they are attached.

22. The olefin metathesis catalyst of claim 1 to 21, wherein R5, R6, R7 and R8 are, independently from each other, selected from hydrogen, hydrogen, methyl, ethyl, propyl, isopropyl, n-butyl, isobutyl, tert.-butyl, methoxy, ethoxy, propoxy, isopropoxy, butoxy, isobutoxy, tert.-butoxy, methylamino, dimethylamino, ethylamino, dimethylamino, propylamino, dipropylamino, isopropylamino, diisopropylamino, butylamino, dibutylamino, isobutylamino, diisobutylamino, tert.-butylamino, di-tert.-butylamino, or electron-withdrawing groups selected from F, Cl, Br and I, trifluormethyl (-CF₃), nitro (-NO₂), formyl (-CHO), C₁-C₆-carboxyl or nitrile (-CN); or wherein R5, R7 and R8 are, independently from each other, selected from hydrogen, hydrogen, methyl, ethyl, propyl, isopropyl, n-butyl, isobutyl, tert.-butyl and R6 is an electron-withdrawing groups selected from F, Cl, Br and I, trifluormethyl (-CF₃), nitro (-NO₂), formyl (-CHO), C₁-C₆-carboxyl or nitrile (-CN).

23. The olefin metathesis catalyst of claim 1 to 22, wherein R11, R12, R13 and R14 are independently hydrogen, methyl, ethyl, propyl, isopropyl, n-butyl, isobutyl, tert.-butyl;
or wherein R11 and R12 are methyl and R13 and R14 are hydrogen; or wherein R11 and R12 are hydrogen and R13 and R14 are methyl.

24. The olefin metathesis catalyst of claim 1 to 23, wherein R1 is hydrogen; R2 is selected from hydrogen, straight chain or branched alkyl groups including C1-C10-alkyl, C1-C10-alkoxy, C1-C10-alkylthio, C1-C10-silyloxy, C1-C10-alkylamino, C6-C 14-aryl, C6-C14-aryloxy, C6-C14-heterocyclic or electron-withdrawing groups selected from halogen atoms, trifluormethyl (-CF3), nitro (-NO2), sulfinyl (-SO-), sulfonyl (-SO2-), formyl (-CHO), C1-C10-carbonyl, C1-C10-carboxyl, C1-C10-alkylamido, C1-C10-aminocarbonyl, nitrile (-CN) or C1-C10-sulfonamide; X1 and X2 are halogen (specifically Cl, Br, I or F) or are independently oxygen or sulfur linked together by a hydrocarbon bridge being part of an aromatic ring or ring system independently;
R3 is adamantyl, , unsubstituted phenyl or substituted phenyl, or R3 is 2,4,6-trimethylphenyl, 2,6-di-iso-propylphenyl, 2-methyl-6-tert-butylphenyl, 2-*iso*-propyl-6-methylphenyl, 2-iso-propyl-phenyl, 2,6-di-ethylphenyl, 2-ethyl-6-methylphenyl or 2-methyl-phenyl;
R3a is methyl, ethyl, n-propyl, or phenyl; or together with R4 can form a five to ten membered cycloalkyl or heterocyclic ring, with the carbon atom to which they are attached; and
R4 is methyl, ethyl, n-propyl, or phenyl; or together with R3a can form a five- to ten-membered cycloalkyl or heterocyclic ring, with the carbon atom to which they are attached;
R11, R12, R13 and R14 are independently hydrogen, unsubstituted C1-C12 alkyl, substituted C1-C12 alkyl, unsubstituted C4-C12 cycloalkyl, substituted C4-C12 cycloalkyl, unsubstituted C5-C24 aryl, substituted C5-C24 aryl, unsubstituted C5-C24 heteroaryl, substituted C5-C24 heteroaryl, unsubstituted C6-C24 aralkyl, substituted C6-C24 aralkyl, unsubstituted C6-C24 heteroaralkyl or substituted C6-C24 heteroaralkyl;
R5, R6, R7 and R8 are, independently from each other, selected from hydrogen, straight chain or branched alkyl groups including C1-C10-alkyl, C1-C10-alkoxy, C1-C10-alkylthio, C1-C10-silyloxy, C1-C10-alkylamino, or electron-withdrawing groups selected from F, Cl, Br and I, trifluormethyl (-CF₃), nitro (-NO₂), sulfinyl (-SO-), sulfonyl (-SO₂-), formyl (-CHO), C₁-C₁₀-carbonyl, C₁-C₁₀-carboxyl, C₁-C₁₀-alkylamido, C₁-C₁₀-aminocarbonyl, nitrile (-CN) or C₁-C₁₀-sulfonamide.
